# EUROPEAN PATENT SPECIFICATION

(11) **EP 2 863 900 B1**
(45) Date of publication and mention of the grant of the patent: **02.11.2016**
(21) Application number: 13739623.0
(22) Date of filing: 21.06.2013
(51) Int. Cl.: A61K 9/20, A61K 31/567

(54) **FORMULATIONS FOR THE PREPARATION OF IMMEDIATE-RELEASE TABLETS FOR ORAL ADMINISTRATION CONTAINING LOW-DOSE MIFEPRISTONE, TABLETS THUS OBTAINED AND THEIR PREPARATION PROCESS**
FORMULIERUNGEN ZUR HERSTELLUNG VON TABLETTEN MIT UNMITTELBARER FREISETZUNG ZUR ORALEN VERABREICHUNG MIT NIEDRIGDOSIERTEM MIFEPRISTON, DARAUS GEWONNENE TABLETTEN UND VERFAHREN ZU DEREN HERSTELLUNG
FORMULATIONS DE PRÉPARATION DE COMPRIMÉS À LIBÉRATION IMMÉDIATE POUR ADMINISTRATION ORALE DE MIFEPRISTONE FAIBLEMENT DOSÉ, COMPRIMÉS AINSI OBTENUS ET LEUR PROCÉDÉ DE PRÉPARATION

(30) Priority: 21.06.2012 IT FI20120127
(43) Date of publication of application: 29.04.2015
(73) Proprietor: VALPHARMA INTERNATIONAL S.P.A., 47864 Pennabilli (IT)
(72) Inventor: VALDUCCI, Roberto, 47039 Savignano sul Rubicone (IT); AVANESSIAN, Serozh, 47827 Villa Verucchio (IT)
(74) Representative: Brighenti, Livio
(86) International application number: PCT/EP2013/063010
(87) International publication number: WO 2013/190097

(56) References cited:
- EP-A1- 1 987 814
- EP-A1- 2 210 585
- CN-A- 101 455 671

## Description

### Field of the invention

The present invention relates to the field of tablets for oral administration in particular tablets containing low doses of active ingredient.

### Background of the invention

Mifepristone, also known as RU486 with chemical formula 11β- [4-(N,N-Dimethylamino)-phenyl-17β-hydroxy-17α-(1-propynyl)-estra-4,9-dien-3-one, is a selective progesterone antagonist at receptor level without progestogenic, oestrogenic, androgenic and anti-oestrogenic activity. This molecule's ability to bind with the progesterone receptor at endometrial level is 5 times higher with respect to that of the progesterone itself. It can be quickly absorbed following oral administration with the appearance of a peak at blood level after just 1 and half hours. It has a long half-life time of around 20 hours (much longer than that of many glucocorticoid agonists), which makes its in vivo clearance very slow. It too is naturally subject to the first passage effect and the blood level of its metabolites after 1-2 hours from oral administration is greater with respect to the original compound. The use of high-dose mifepristone has proved effective as emergency post-coital contraceptive treatment for hospital use in combination with misoprostol by oral route or prostaglandin E₁ by vaginal route. There are prior art studies that have demonstrated the efficacy of mifepristone for the treatment of other diseases such as leimyoma, myoma, uterine fibrosis, endometriosis, adenomyosis and related disorders.

Eisinger et al (2003) have demonstrated that the daily oral administration of a low-dose of Mifepristone (5 mg) for a period of 6 months has effects comparable with those of higher doses of Mifepristone in the treatment of uterine fibroma with reduction of the related side effects.

Intravaginal tablet formulations that offer the same advantages as the oral formulations but with a lower dose and thus a reduction of the possible appearance of side effects are described in patents US 2011 /0208118 A1, WO 2009/037704 A1 and EP 2 197 415 A0.

Other studies have demonstrated the efficacy of this medicinal product in the treatment of Amyotrophic Lateral Sclerosis (patent US 2011 /0166115 A1, WO 2010/002901 and EP 2 306 830 A0) and of Cushing's syndrome in combination with an inhibitor of cortisone synthesis such as Metyrapone or mitotane (US 2010/0261693 A1, WO 2009/050136 A2 and EP 2 211 845 A0).

EP 2 210 585 1 discloses 3 different mifepristone compositions formulated as tablets for the treatment of uterine fibroids, the compositions are given in % (w/w). The first composition consists of: mifepristone 1.02 %; MCC 62.98%; polyvinylpyrrolidone 10%; Mg stearate 2%; HPMC 14% and crospovidone 10%; The second composition consists of: mifepristone 1.02 %; MCC 46.98%; polyvinylpyrrolidone 10%; Mg stearate 2%; HPMC 14%; lactose 16% and croscarmellose Na 10%; The third composition consists of: mifepristone 1.02 %; MCC 54.98%; polyvinylpyrrolidone 10%; Mg stearate 2%; HPMC 12%; lactose 5% and croscarmellose Na 15%.

It should also be borne in mind that mifepristone, being equipped with a high activity, requires special conditions for the safe processing thereof, which must take place in suitable premises and with equipment capable of allowing processing in containment conditions.

It is thus evident, from what has been said above, how useful it would be to have low-dose mifepristone formulations in the form of immediate-release tablets for oral administration.

### Summary of the invention

The present invention relates to formulations containing a low dose of mifepristone for the preparation of immediate-release tablets, as defined in the claims, to be used in the treatment of uterine fibroma in particular.

### Detailed description of the invention

The present invention allows the above-mentioned problems to be resolved thanks to a formulation and to a production process that does not require special procedures, allows the problems due to the handling of the mifepristone to be reduced and thus allows immediate-release tablets for oral administration containing a low dose of the active ingredient, to be effectively made available.

The use of microcrystalline cellulose containing moisture, water, between 1 - 10 weight %, which indeed allows the formation of the necessary microgranulate premix, this granulation makes the mixture smooth-flowing and easily compressible and reduces the segregation of the individual components of the mixture and this allows tablets with an excellent uniformity of content to be obtained.

The components of the formulation are present in the following amounts: Mifepristone 6% w/w; Microcristalline Cellulose (with a water content 1-10% w/ w) 85.7% w/w; Polyvinylpyrrolidone 4% w/w; Croscarmellose Sodium 3% w/w; Magnesium Stearate 1 % w/w; Anhydrous Colloidal Silica 0.3 % w/w.

According to the invention, the tablets are produced by the formation of a premix wherein the active ingredient comes to be mixed with an equal amount of microcrystalline cellulose and is sieved.

In the meantime, the remaining microcrystalline cellulose, binder and disintegrant are sieved and mixed together. To this mixture is added and mixed the premix containing the active ingredient. Lastly, the lubricants and glidants are added and such a mixture is compressed.

Using the above-described components and process, it is possible to obtain immediate-release, low-dose Mifepristone tablets for oral administration that guarantee the uniformity of content requirements with excellent uniformity of content.

It is thus possible to easily obtain, and with fewer risks, low-dose mifepristone tablets that guarantee therapeutic efficacy in the treatment of uterine fibroma with fewer side effects.

### Example

Formulation of low-dose, immediate-release mifepristone tablets with excellent uniformity of content.

For a batch of 1,000,000 tablets 5 kg of raw material 71.417 kg of microcrystalline cellulose, 3.333 kg of polyvinylpyrrolidone, 2.5 kg of croscarmellose sodium, 0.833 kg of magnesium stearate and 0.250 kg of anhydrous colloidal silica were used.

### a) Preparation of the Premix

The mifepristone and the microcrystalline cellulose are mixed together and the mixture is sieved.

### b) Mixing of the excipients

The remaining microcrystalline cellulose, polyvinyl pyrrolidone and croscarmellose sodium are mixed together.

### c) Mixing of the excipients with the active premix

To the excipient mixture is added the premix containing the active ingredient and mixed in BIN for 10 minutes at 8 rpm.

### d) Addition of lubricants and glidants

The magnesium stearate and microcrystalline silica are sieved and added to the BIN to be mixed with the remaining components.

### e) Compression of the mixture.

The mixture is compressed to obtain tablets having a 6mm diameter and average weight of 80 mg with active ingredient content equal to 5 mg/TBL.

## Claims

1. Immediate-release tablets for oral administration consisting of:
Mifepristone 6% w/w;
Microcristalline Cellulose (with a water content 1-10% w/w) 85.7% w/w;
Polyvinylpyrrolidone 4% w/w;
Croscarmellose Sodium 3% w/w;
Magnesium Stearate 1% w/w;
Anhydrous Colloidal Silica 0.3 %.

2. Tablets according to claim 1 for use in a method of treatment of uterine fibroma.

3. A process for the preparation of immediate-release tablets for oral administration comprising a direct compression step of a formulation according to claim 1.

4. A process according to claim 3, wherein:
a) a premix is prepared by mixing the active ingredient with an equal amount of microcrystalline cellulose and sieved;
b) the remaining microcrystalline cellulose, the binder and disintegrant are sieved and mixed together
c) the mixture prepared in point (b) is added to the premix containing the active ingredient and lubricants and lastly glidants are added
d) the aforementioned mixture is compressed into tablets.

## Patentansprüche

1. Tabletten mit unmittelbarer Freisetzung zur oralen Verabreichung, zusammengesetzt aus:
Mifepriston 6 Gew.-%
Mikrokristalline Zellulose (mit einem Wassergehalt von 1 - 10 Gew.-%) 85,7 Gew.-% Polyvinylpyrrolidon 4 Gew.-%
Croscarmellose-Natrium 3 Gew.-%
Magnesium-Stearat 1 Gew.-%
Anhydrische Kolloid-Kieselerde 0,3 Gew.-%

2. Tabletten gemäß Anspruch 1 zur Verwendung in einem Behandlungsverfahren bei Gebärmutterfibromen.

3. Verfahren zur Herstellung von Tabletten mit unmittelbarer Freisetzung zur oralen Verabreichung, das einen direkten Schritt der Tablettierung der Rezeptur gemäß Anspruch 1 aufweist.

4. Verfahren gemäß Anspruch 3, wobei:
a) ein Vorgemisch hergestellt wird, indem die aktive Ingredienz mit einer gleichen Menge mikrokristalliner Zellulose vermischt und durchgesiebt wird;
b) die restliche mikrokristalline Zellulose, das Bindemittel und das Desintegrationsmittel durchgesiebt und zusammengemischt werden.
c) die Mischung aus (b) zum Vorgemisch, das die aktive Ingredienz enthält, hinzugefügt wird sowie Gleitmittel und schließlich Fließmittel hinzugefügt werden.
d) die vorgenannte Mischung zu Tabletten gepresst wird.

## Revendications

1. Comprimés à libération immédiate pour une administration orale constitués de :
mifépristone 6 % p/p ;
cellulose microcristalline (avec une teneur en eau de 1 à 10 % p/p) 85,7 % p/p ;
polyvinylpyrrolidone 4 % p/p ;
croscarmellose sodique 3 % p/p ;
stéarate de magnésium 1 % p/p ;
silice colloïdale anhydre 0,3 %.

2. Comprimés selon la revendication 1 pour une utilisation dans un procédé de traitement du fibrome utérin.

3. Procédé pour la préparation de comprimés à libération immédiate pour une administration orale comprenant une étape de compression directe d'une formulation selon la revendication 1.

4. Procédé selon la revendication 3, dans lequel :
a) un prémélange est préparé en mélangeant le principe actif avec une quantité égale de cellulose microcristalline et tamisé ;
b) la cellulose microcristalline restante, le liant et l'agent de désagrégation sont tamisés et mélangés ensemble
c) le mélange préparé au point (b) est ajouté au prémélange contenant le principe actif et des lubrifiants et finalement les agents de glissement sont ajoutés
d) le mélange mentionné précédemment est compressé en comprimés.
